(19) Europäisches Patentamt
European Patent Office
Office européen des brevets

(11) EP 1 254 955 A1

(12) **EUROPEAN PATENT APPLICATION**

(43) Date of publication:
**06.11.2002 Bulletin 2002/45**

(51) Int Cl.[7]: **C12N 15/11**, C07D 311/10

(21) Application number: **02252856.6**

(22) Date of filing: **23.04.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.04.2001 JP 2001124452**

(71) Applicant: **RIKEN**
**Wako-shi, Saitama 351-0198 (JP)**

(72) Inventors:
- **Okamoto, Hitoshi**
  **Wako-shi, Saitama 351-0198 (JP)**
- **Ando, Hideki**
  **Wako-shi, Saitama 351-0198 (JP)**
- **Furuta, Toshiaki**
  **Narashino-shi, Chiba 275-0004 (JP)**

(74) Representative:
**Denison, Christopher Marcus et al**
**Mewburn Ellis**
**York House**
**23 Kingsway**
**London WC2B 6HP (GB)**

(54) **A method for regulating gene expression**

(57) A target gene can be expressed site-specifically and with desired timing without damaging the cell itself or inducing mutation in a nucleic acid molecule within the cell. Expression of the above target gene is suppressed by allowing a caging agent represented by the following formula (1) to bind to a nucleic acid molecule containing the target gene; and the suppression of expression of the above target gene is cancelled by detaching the caging agent from the above nucleic acid molecule by UV-illumination.

FIG.1

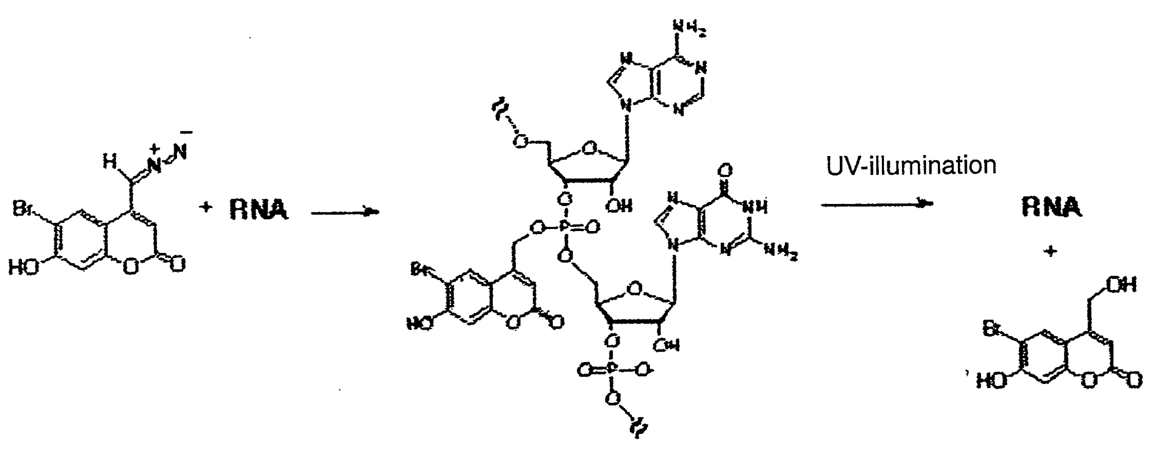


EP 1 254 955 A1

## Description

The present invention relates to a method for regulating gene expression which can activate or suppress the expression of a target gene to be analyzed with desired timing, and in desired tissues and cells.

A known method for expressing a gene to be analyzed with desired timing is a method using 1-(4,5-dimethoxy-2-nitrophenyl) diazoethane (DMNPE) as a caging group (W. Todd Monroe (1999), Jour. Biol. Chem. 274, 20895-20900). This method involves allowing binding of a plasmid DNA molecule containing luciferase gene to DMNPE to lower the expression efficiency, followed by illumination with ultraviolet rays with desired timing to free DMNPE from the plasmid DNA, to thus recover the expression efficiency of the luciferase gene.

That is, this method involves transfecting a DNA molecule bound to DMNPE into a cell, and then illuminating the cell with ultraviolet rays at a wavelength of 365 nm, about 10 cm away from the cell, for about 20 min. UV-illumination to the cell separates DMNPE from the DNA molecule intracellularly. According to this method, the expression efficiency which is suppressed at the transcription stage of the DNA molecule is improved by detachment of DMNPE from the DNA molecule, so that luciferase can be expressed.

With this method, a target gene can be expressed site-specifically and with desired timing in a transfected cell. However, as the duration of UV-illumination in this method is long, being about 20 min, it may induce mutation in transfected cells, or kill a large part of the cells. Therefore, a problem with this method is that it is impractical.

The present invention has been achieved in view of the above-described circumstances. An object of the present invention is to address the above-mentioned problems, and preferably to provide a method for regulating gene expression which enables expression of a target gene site-specifically and with desired timing, without damaging the cell itself or inducing mutation in a nucleic acid molecule within a cell.

As a result of thorough studies to achieve the above-mentioned purpose, we have clarified that binding of a caging agent, such as 6-bromo-4-diazomethyl-7-hydroxycoumarin, to a nucleic acid molecule can lower the expression efficiency of the nucleic acid molecule, and UV-illumination can free the caging agent from the nucleic acid molecule, and we have completed the present invention by finding that expression of a target gene can be regulated by controlling binding of a caging agent to a nucleic acid molecule containing a target gene and freeing of the caging agent from the nucleic acid molecule.

That is, the present invention is a method for regulating gene expression which comprises illuminating with ultraviolet rays a nucleic acid molecule to which a caging agent comprising a compound represented by the following formula (1) has been previously bound.

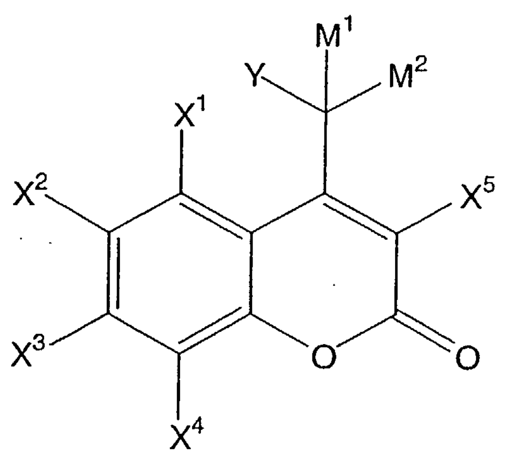

(1)

(wherein $X^1$, $X^2$, $X^3$, $X^4$ and $X^5$ are -H, hydroxyl group, substituted alkoxy group, unsubstituted alkoxy group, -OC(O)$R^1$ group, -$NH_3$ group, -$NR^1R^2$ group, -$R^1$, -F, -Cl, -Br, -I, -COOH, -$NO_2$, -C(=O)NH$R^1$, -CN, -CHO, -C(=O)$R^1$ or -$SO_3$H, respectively. Y and $M^2$ are -H, -$R^1$, -$NR^1R^2$, -C(=O)$R^1$ or -COOH. $M^1$ is -H, -Cl, -Br, -I or -$OSO_2R^1$ or -OH, or $M^1$ and $M^2$ form =$N_2$ or =NNH$R^1$. Here, $R^1$ and $R^2$ are substituted functional groups or unsubstituted functional groups, respectively, selected from the group consisting of an alkyl group having a carbon number of 1 to 20, an alkenyl group having a carbon number of 2 to 20, an alkynyl group having a carbon number of 2 to 20, an alkoxy group having a carbon number of 1 to 20, a thioalkoxy group having a carbon number of 1 to 20, an alkylsulfonyl group having a carbon number of 1 to 20, an allylsulfonyl group having a carbon number of 4 to 16, a heteroalkyl group having a carbon

number of 2 to 20, a heteroalkenyl group having a carbon number of 2 to 20, a cycloalkyl group having a carbon number of 3 to 8, a cycloalkenyl group having a carbon number of 3 to 8, an allyl group having a carbon number of 4 to 16, a heteroallyl group having a carbon number of 4 to 16, and a heterocyclyl group having a carbon number of 2 to 30.)

The method for regulating gene expression according to the present invention may be a method which involves transfecting a nucleic acid molecule to which the above caging agent has been bound into a host cell, and illuminating the transfected host cell with the above ultraviolet rays. The above host cell preferably allows penetration of long-wave ultraviolet rays.

Further, the present method may be a method which involves illuminating with the ultraviolet rays a certain region of the above host cell, allowing expression of a target gene only in the certain region. Here, the above certain region may be the entire host cell or one part thereof.

The present invention is described in detail below.

In the method for regulating gene expression according to the present invention, first, a nucleic acid molecule containing a gene to be regulated is bound to a caging agent represented by the following formula (1), so that the expression of the gene is suppressed.

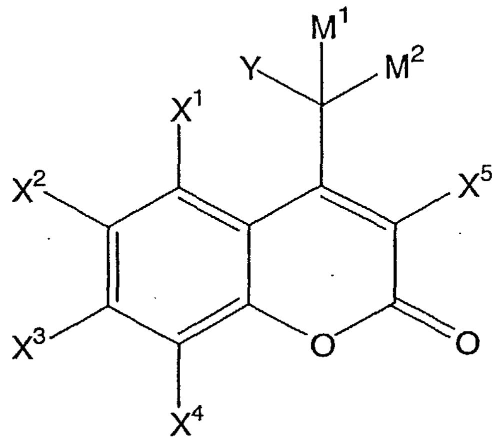

(1)

(wherein $X^1$, $X^2$, $X^3$, $X^4$ and $X^5$ are -H, hydroxyl group, substituted alkoxy group, unsubstituted alkoxy group, -OC(O)$R^1$ group, $-NH_3$ group, $-NR^1R^2$ group, $-R^1$, -F, -Cl, -Br, -I, -COOH, $-NO_2$, $-C(=O)NHR^1$, -CN, -CHO, $-C(=O)R^1$ or $-SO_3H$, respectively. Y and $M^2$ are -H, $-R^1$, $-NR^1R^2$, $-C(=O)R^1$ or -COOH. $M^1$ is -H, -Cl, -Br, -I or $-OSO_2R^1$ or -OH, or $M^1$ and $M^2$ form $=N_2$ or $=NNHR^1$. Here, $R^1$ and $R^2$ are substituted functional groups or unsubstituted functional groups, respectively, selected from the group consisting of an alkyl group having a carbon number of 1 to 20, an alkenyl group having a carbon number of 2 to 20, an alkynyl group having a carbon number of 2 to 20, an alkoxy group having a carbon number of 1 to 20, a thioalkoxy group having a carbon number of 1 to 20, an alkylsulfonyl group having a carbon number of 1 to 20, an allylsulfonyl group having a carbon number of 4 to 16, a heteroalkyl group having a carbon number of 2 to 20, a heteroalkenyl group having a carbon number of 2 to 20, a cycloalkyl group having a carbon number of 3 to 8, a cycloalkenyl group having a carbon number of 3 to 8, an allyl group having a carbon number of 4 to 16, a heteroallyl group having a carbon number of 4 to 16, and a heterocyclyl group having a carbon number of 2 to 30.)

The caging agent shown by formula (1) maybe prepared as follows. First, a substituted phenol is prepared as a starting substance. Then, a coumarin derivative is synthesized by reaction of the substituted phenol and a substituted or unsubstituted acetoacetic ester. Next, a desired substituent is introduced to an alkyl group at position 4 of the coumarin derivative, so that the caging agent comprising the compound represented by formula (1) can be prepared.

For example, when introducing a diazo group to the alkyl group at position 4, the alkyl group at position 4 of a coumarin derivative is oxidized and a carbonyl group is introduced thereto, and this is then condensed with a substituted hydrazine, so as to convert the carbonyl group into a hydrazone. The obtained hydrazone derivative is treated with bases so that the diazo group can be introduced to the alkyl group at position 4.

Further, for example, when a halogen substituent is introduced to the alkyl group at position 4, a 4-halogenated acetoacetic ester is used as a substituted acetoacetic ester. A coumarin derivative in which a halogen substituent has been introduced to the alkyl group at position 4 can be synthesized by reaction of the 4-halogenated acetoacetic ester and a substituted phenol.

Furthermore, for example, when a hydroxyl group is introduced to the alkyl group at position 4, after a halogen

substituent is introduced to the alkyl group at position 4 as described above, the halogen substituent is hydrolyzed. Thus, a coumarin derivative in which a hydroxyl group has been introduced to the alkyl group at position 4 can be synthesized.
The caging agent is preferably prepared as a solution of the caging agent at a concentration of 40 to 80 μg/μl in a solvent, such as dimethyl sulfoxide.

Examples of a nucleic acid molecule subjected to regulation of expression in the present method include plasmid DNA, genomic DNA, synthetic DNA synthesized by PCR or the like, cDNA, mRNA, antisense RNA and cyclic nucleotides. That is, nucleic acids to be regulated may be either DNA or RNA, so far as it contains a gene subjected to regulation of expression. Further, the gene may be a gene encoding a protein with a known function, or may be a gene encoding a protein with unknown function. Furthermore, examples of a nucleic acid molecule include not only a gene subjected to regulation of expression, but also those containing promoter regions or the like which regulate expression of the gene. A nucleic acid molecule is prepared as, for example, a solution of the nucleic acid molecule at a concentration of 50 to 500 ng/μl in a solvent of DMSO or the like.

Moreover, to bind a caging agent to the above nucleic acid molecule, a solution of the caging agent may be mixed with a solution of the nucleic acid molecule in, for example, equivalent amounts (5 μl : 5 μl), and then, for example, the mixture is allowed to stand at room temperature (20 to 26°C) for 15 to 120 min. Thus, the present method enables a caging agent to bind to a nucleic acid molecule by mixing a solution of a nucleic acid molecule with a solution of a caging agent, and then allowing the mixture to stand. Further, at this time, preferably the reaction solution is subjected to column chromatography or the like according to a standard technique, the unreacted caging agent is removed, and then nucleic acid molecules with the caging agent bound thereto are purified.

When the caging agent is bound to a nucleic acid molecule, expression of a gene contained in the nucleic acid molecule is suppressed. For example, when DNA is used as a nucleic acid molecule, transcription of the nucleic acid molecule is suppressed within a host having a transcription system, so that expression of the gene is suppressed. In addition, when mRNA is used as a nucleic acid molecule, translation of the nucleic acid molecule is suppressed within a host having a translation system, so that expression of the gene is suppressed.

Next, the present method allows the caging agent to detach from the above nucleic acid molecule by UV-illumination. Therefore, suppression of the expression of the above target gene can be canceled.

To activate a target gene at the cellular level, a nucleic acid molecule with the caging agent bound thereto is transfected into a host cell, and then the host cell is illuminated with ultraviolet rays. That is, for a nucleic acid molecule with the caging agent bound thereto, expression of the gene is suppressed within a host cell. However, UV-illumination causes the caging agent to detach from the nucleic acid molecule, so that expression of the gene is activated.

Further, when antisense RNA or an antisense oligonucleotide is used as a nucleic acid molecule, the caging agent is bound to the antisense RNA or the like, so that the expression-suppressing activity of the antisense RNA or the like can be suppressed. That is, the caging agent is bound to, for example, antisense RNA corresponding to a certain gene, so that the gene in a host cell can be expressed. Then, UV-illumination enables detachment of the caging agent from the antisense RNA, thereby promoting the activity of the antisense RNA which suppresses the expression of the above gene. Therefore, the use of the caging agent of the present invention can inactivate a certain gene, time period-specifically and/or tissue-specifically.

Host cells used herein may be any cell which allows transmission of illuminated ultraviolet rays and supply of the energy of ultraviolet rays to the transfected nucleic acid molecule. Examples of host cells include embryo of zebrafish, human cells, fruit-fly cells, nematode cells, embryo of Xenopus at the early developmental stage, and cultured cells or the like in monolayer form derived from various organisms.

A known standard technique may be used as a technique to transfect a nucleic acid molecule into a host cell. When DNA is used as a nucleic acid molecule, examples of a transfection technique include a technique using calcium phosphate, a technique using DEAE-dextran, a technique using electroporation, a technique for transfection mediated by liposome, a microinjection technique and the like. Further, when RNA is used as a nucleic acid molecule, examples of a transfection technique include a microinjection technique, a technique using electroporation, a technique for transfection mediated by liposome and the like. When embryos of zebrafish are used, a nucleic acid molecule to which a caging agent is bound, for example, an mRNA with a caging agent bound thereto, can be transfected by the microinjection technique.

Ultraviolet rays used in the present method means an electromagnetic wave at a wavelength longer than that of x-rays but shorter than that of visible rays, ranging from approximately 1 to 400 nm. Particularly, in the present method, ultraviolet rays on the long wavelength side, for example, ultraviolet rays at a wavelength ranging from 350 to 380 nm are preferably used. Further, ultraviolet rays at a wavelength of about 365±6 nm are preferably used. Furthermore, in the present method, especially when a fluorescent microscor provided with a 100 W mercury lamp (for example, Zeiss, Axioplan II, objective lens with magnification of 10- to 40-fold) is used, UV-illumination is performed preferably for 0.1 to 5.0 sec, more preferably, 0.5 to 2.0 sec, and most preferably 0.5 to 1.0 sec.

The caging agent shown by the above formula (1) detaches from a nucleic acid molecule when illuminated

with ultraviolet rays at a wavelength of about 365 ± 6 nm for about 0.1 to 5.0 sec. More than 5.0 sec illumination with ultraviolet rays at a wavelength of about 365 ± 6 nm may induce mutation in the nucleic acid molecule and may damage the host cells. Less than 0.1 sec illumination with ultraviolet rays at a wavelength of 365 ± 6 nm may result in incomplete detachment of the caging agent from the nucleic acid molecule.

The present method can activate gene expression in the entire host cell or a part thereof by illuminating with ultraviolet rays the entire host cell or a part thereof. When illuminating the entire host cell with ultraviolet rays, the host cell is positioned facing a UV-illuminating means, such as a mercury lamp. Accordingly, a target gene can be activated in the entire host cell. Furthermore, when illuminating a part of a host cell with ultraviolet rays, ultraviolet rays irradiated from a UV-illuminating means, such as a mercury lamp, may be processed into a spot light by an optical system including an objective lens and the like, thereby spot-illuminating with ultraviolet rays only a certain region of the host cell. Thus, a target gene only in a certain region of a host cell can be activated by spot-illumination.

Particularly, when the compound of the above formula (1) is used as a caging agent, as described above, supply of very small amount of energy can ensure detachment of the caging agent from a nucleic acid molecule. At this time, the energy to be supplied to the nucleic acid molecule and the caging agent is, for example, 100 $mJ/cm^2$ or less.

Therefore, when the compound of the above formula (1) is used as a caging agent, UV-illumination may not induce mutation in a nucleic acid molecule or disrupt a host cell itself. Thus, the present method is particularly preferred when gene expression in a host cell is analyzed.

In the present method, a nucleic acid molecule with a caging agent bound thereto may be illuminated in vitro with ultraviolet rays. That is, the present method can suppress in vitro expression of a gene contained in the nucleic acid molecule with the caging agent bound thereto, and then improve the expression efficiency of the gene by UV-illumination at a certain time period after suppression. Examples of in vitro regulation of gene expression include in vitro regulation of transcription at the transcriptional stage using DNA as a nucleic acid molecule and in vitro regulation of translation at the translational stage using mRNA as a nucleic acid molecule.

The above-described method enables in vivo or in vitro expression of a gene with unknown function under spatially and/or temporally arbitrary conditions. For example, a gene with unknown function can be specifically expressed at a desired site of a host cell, or at a desired time, or at a desired site and time. Therefore, the above-described method can be used when analyzing an unknown function of a gene.

Further, the above-described method can also be applied to a screening method for identifying a novel gene. The screening method involves preparing a full length cDNA library, apportioning the cDNA library in pools of, for example, 100 clones each, to prepare multiple gene pools, synthesizing in vitro mRNA for each gene pool, allowing the above caging agent to bind to the synthesized mRNA, and then injecting the gene pool containing the mRNA with the caging agent bound thereto into a host cell.

For example, to identify a gene to develop an eye of a zebrafish, first, multiple gene pools are prepared from the cDNA library of the zebrafish, and then mRNA is synthesized for each gene pool. Then, the multiple gene pools are separately transfected into different embryos of zebrafish. Thereafter, regions of an embryo other than the region developing into the eye are respectively illuminated with ultraviolet rays. Development of the eye at a site illuminated with ultraviolet rays indicates that a gene for development of the eye has been contained in the injected gene pool. Subsequently, screening is further performed using the gene pool containing the desired gene, so that a gene for development of the eye can be identified.

With the screening method using the present invention, the above-described screening performed for 1,000 gene pools enables screening of a total of 100,000 cDNAs, when 100 cDNA clones are contained in each gene pool.

## BRIEF DESCRIPTION OF THE DRAWINGS

Figure 1 shows a reaction for binding a caging agent to mRNA; and a reaction for detaching the caging agent bound to the mRNA.

Figure 2 is a photograph of electrophoresis showing changes in electrophoretic mobility resulting from binding and detachment of a caging agent to and from mRNA.

Figure 3 shows characteristic diagrams showing changes in the absorption wavelength of the mRNA with a caging agent bound thereto.

Figure 4 is a photograph of 12% SDS polyacrylamide gel electrophoresis showing suppression of protein synthesis due to binding of a caging agent.

Figure 5 shows microphotographs showing the presence or absence of expression of mRNA containing GFP gene within embryos of zebrafish.

Figure 6 shows microphotographs showing the enlarged view of typical examples of the embryos shown in Fig. 5.

Figure 7 is a schematic diagram of embryos schematically showing the sites illuminated with ultraviolet rays.

Figure 8 shows microphotographs showing GFP fluorescence when an embryo was spot-illuminated with ultraviolet rays.
Figure 9 shows microphotographs showing staining with GFP fluorescence and anti-GFP antibody when an embryo was spot-illuminated with ultraviolet rays.
Figure 10 shows microphotographs showing the effect of overexpression of En2 mRNA.
Figure 11 is a characteristic figure quantitatively showing the effect of overexpression of En2 mRNA.
Figure 12 shows microphotographs of an embryo in which both mRNA containing GFP gene and mRNA containing En2 gene were co-expressed.
Figure 13 shows microphotographs showing the results of immunohistochemical staining using 4D9 performed for confirming expression of En2.
Figure 14 shows microphotographs showing the results of in situ hybridization using En2 probe which was performed to confirm stabilization of mRNA by a caging agent.
Figure 15 shows microphotographs showing the results of in situ hybridization using gene markers.
Figure 16 shows microphotographs showing the results of double in situ hybridization for otx2 and pax2.
Figure 17 shows microphotographs showing the results of double in situ hybridization for otx2 and krox20.

EXAMPLES

The present invention will be further described in detail below using examples, but the technical scope of the present invention is not limited by these examples.

[Example 1]

In Example 1, expression (translation) of mRNA containing a gene (hereinafter, GFP gene) encoding a green fluorescent protein is regulated in vitro by applying the above method for regulating gene expression according to the present invention.

<u>mRNA preparation method</u>

To obtain mRNA containing GFP gene, first, a full-length cDNA of GFP gene was cloned into a pCS2 vector as follows. Specifically, first, pEGFP plasmid (CLONTECH) was cleaved with restriction enzymes BamH I and EcoR I, and then a DNA fragment containing a gene encoding EFGP was cleaved out from the pEGFP plasmid. The obtained DNA fragment was inserted between BamH I site and EcoR I site of a pCS2+ vector, thereby obtaining a cDNA plasmid.
Next, mRNA was synthesized using the obtained cDNA plasmid as a template, mMESSAGE mMACHINE (Ambion, U.S.A.), and SP6 RNA polymerase (Ambion). Thus, mRNA containing GFP gene was obtained. The obtained mRNA was dissolved in RNase-free water at a concentration of 1 μg/μl, and then stored at -80°C.

<u>mRNA caging</u>

A caging agent was bound to mRNA containing GFP gene by the method described below. A caging agent used in this example had the structural formula represented by the following formula.

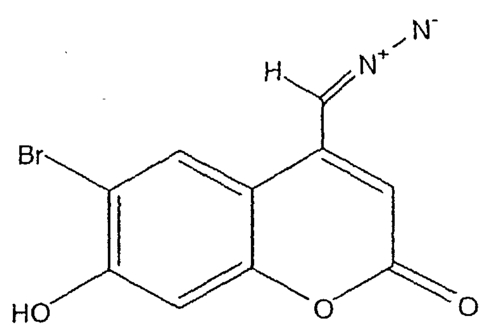

This caging agent was 6-bromo-7-hydroxy-4-diazomethylcoumarin, and was prepared according to the following reaction formula.

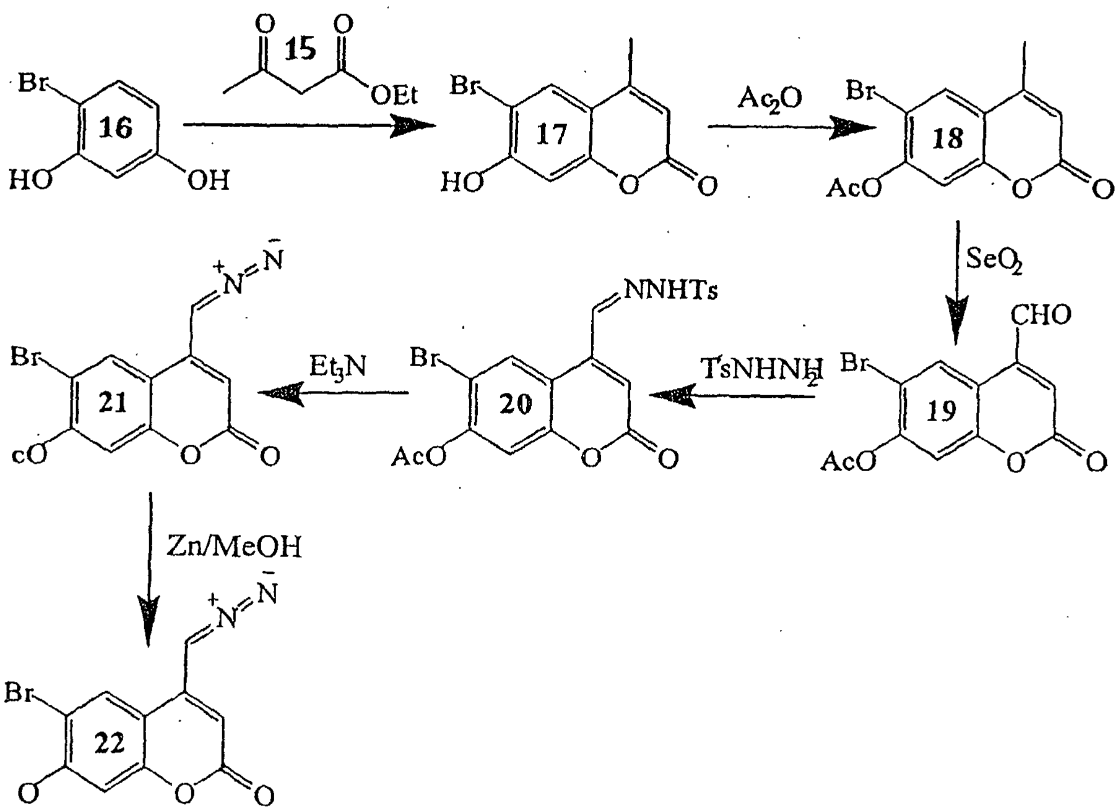


Specifically, 6-bromo-7-hydroxy-4-methylcoumarin (17), 7-acetoxy-6-bromo-4-methylcoumarin (18), 7-acetoxy-6-bromo-4-formylcoumarin (19), 7-acetoxy-6-bromo-4-formylcoumarin tosylhydrazone (20), 7-acetoxy-6-bromo-4-diazomethylcoumarin (21), and finally the caging agent, 6-bromo-7-hydroxy-4-diazomethylcoumarin (22), were synthesized sequentially.

To synthesize 6-bromo-7-hydroxy-4-methylcoumarin (17), first, 936.1 mg (4.953 mmol) of a 4-bromoresorcinol (16) solution, 5 ml of concentrated sulfuric acid and ethyl-4-acetoacetate (15) were mixed at room temperature for 6 days. Next, the reaction mixture was poured into ice water, and then mixed for 2 hours, thereby obtaining a precipitate. Then, the precipitate was collected by filtration, and then washed with cold-water, followed by vacuum drying in the presence of $P_2O_5$. Thus, 6-bromo-7-hydroxy-4-methylcoumarin (17) with a yield of 847.6 mg (2.928 mmol, yield 59.1%) was synthesized.

To synthesize 7-acetoxy-6-bromo-4-methylcoumarin (18), first, 453.1 mg (1.776 mmol) of a 6-bromo-7-hydroxy-4-mechylcoumarin (17) solution and 545 ml (3.9 mmol) of triethylamine contained in dry acetonitrile were added to 255 ml (3.59 mmol) of acetylchloride. Subsequently, the reaction mixture was mixed at room temperature for 15 hours, and then the reaction was ceased with 1N hydrochloric acid. Next, chloroform was added to the reaction mixture, separating the organic phase. The separated organic phase was washed with a saturated $NaHCO_3$ aqueous solution and saturated brine, and then dried ($MgSO_4$). Then the solvent was evaporated. Thus, crude 7-acetoxy-6-bromo-4-methylcoumarin (18) with a yield of 404.9 mg (1.363 mmol, yield 76.7%) was synthesized.

To synthesize 7-acetoxy-6-bromo-4-formylcoumarin (19), first, a mixture containing 404.9 mg (1.363 mmol) of 7-acetoxy-6-bromo-4-methylcoumarin (18) and 239.2 mg (2.156 mmol) of selenium oxide contained in 15 ml of m-xylene were mixed at room temperature for 21 hours while refluxing. Next, the reaction mixture was cooled and the solvent was evaporated. Then, purification was performed with column chromatography. Thus, 7-acetoxy-6-bromo-4-formylcoumarin (19) with a yield of 287.0 mg (0.9226 mmol, yield 67.7%) was synthesized.

To synthesize 7-acetoxy-6-bromo-4-formylcoumarin tosylhydrazone (20), first, 61.6 mg (0.3308 mmol) of p-toluenesulfenylhydrazide was added to 4 ml of an ethanol solution containing 101.8 mg (0.3273 mmol) of 7-acetoxy-6-bromo-4-formylcoumarin (19) while stirring the solution. 17 hours later, the obtained precipitate was collected by filtration, washed, and then dried under vacuum. Thus, 7-acetoxy-6-bromo-4-formylcoumarin tosylhydrazone (20) with a yield of 103.2 mg (0.2153 mmol, yield 65.8%) was synthesized.

To synthesize 7-acetoxy-6-bromo-4-diazomethylcoumarin (21), first, a mixture containing 103.2 mg (0.2153 mmol) of 7-acetoxy-6-bromo-4-formylcoumarintosylhydrazone (20) was mixed with 15 ml of a methanol solution containing 45 ml (0.32 mmol) of triethylamine under $N_2$ atmosphere at room temperature for 5.5 hours. Then, the obtained

yellow precipitate was collected by filtration, washed with a minimum amount of methanol, and then dried under vacuum. Thus, 7-acetoxy-6-bromo-4-diazomethylcoumarin (21) with a yield of 43.0 mg (0.133 mmol, yield 62%) was synthesized.

To synthesize 6-bromo-7-hydroxy-4-diazomethylcoumarin (22) to be used as a caging agent, first, a mixture containing 43.0 mg (0.133 mmol) of 7-acetoxy-6-bromo-4-diazomethylcoumarin (21) was mixed with 10 ml of a methanol solution containing 154.6 mg (2.36 mmol) of zinc under $N_2$ atmosphere at room temperature for 75 hours. After zinc was removed by filtration, the solution was completely washed with methanol, and the solvent in the obtained organic phase was evaporated. Thus, 6-bromo-7-hydroxy-4-diazomethylcoumarin (22) with a yield of 37.5 mg (0.133 mmol, yield 100%) was synthesized.

6-bromo-7-hydroxy-4-diazomethylcoumarin (22) synthesized as described above, that is, a caging agent, was dissolved in DMSO, and then subjected as a Bhc-diazo solution (60 μg/μl) to the following experiment.

mRNA in 5 μl of RNase-free water was precipitated, re-suspended in 5 μl of DMSO, and then mixed with 5 μl of the Bhc-diazo solution (60 μg/μl). The reaction mixture was allowed to stand at room temperature for 180 min, and then subjected to column chromatography using Sephadex G50 which had been allowed to swell in DMSO and equilibrated, thereby removing free Bhc-diazo from the mixture.

The mRNA with the caging agent bound thereto was contained in the first 500 μl of fraction obtained by column chromatography. An equal volume of isopropanol and 0.1 volume of 10 M ammonium acetate were added to the elution fraction, so as to precipitate the mRNA. Subsequently, the precipitate was washed with 70% ethanol and dried, thereby obtaining the mRNA with the caging agent bound thereto. Then, the mRNA was dissolved in RNase-free water to have a concentration of 1 μg/μl, so that an mRNA solution was obtained. Figure 1 shows a reaction to bind the caging agent to mRNA and a reaction to separate the caging agent bound to mRNA.

Confirmation of binding of caging agent to mRNA

I. Electrophoresis

Part of the obtained mRNA solution was apportioned into three samples, 1 to 3. Sample 1 was illuminated with ultraviolet rays at a wavelength of 365 nm for 10 sec using a 100W mercury lamp at 10% of the maximum intensity. Sample 2 was illuminated with ultraviolet rays at a wavelength of 365 nm for 20 sec under the same conditions. Sample 3 was not illuminated with ultraviolet rays. Further, a solution of mRNA with no caging agent bound thereto was prepared as sample 4.

These samples 1 to 4 were subjected to 1.0% agarose gel electrophoresis. Figure 2 shows the results. In Fig. 2, lane 1 denotes sample 4, lane 2 denotes sample 3, lane 3 denotes sample 1, and lane 4 denotes sample 2.

Compared to lane 1, lane 2 showed a smear on a side corresponding to the fragment size smaller than that of the band of mRNA. Lanes 3 and 4 showed reduced smears and recovery of the intensity of the mRNA band. These results suggest that smears observed in lanes 2 to 4 were not degradation products, but rather were produced by conformational changes in mRNA as a result of covalent attachment of the caging agent to mRNA.

II. Spectrophotometric analysis

Binding efficiency of the caging agent to mRNA was verified by spectrophotometric analysis. Samples used herein were prepared by respectively diluting 1:150 in KMOPS buffer (10 mM (N-morpholino) propanesulfonic acid, 150 mM KCl, 5 mM $MgCl_2$, pH 7.2) 1 μg of mRNA which had been allowed to stand at room temperature for 1 hour for binding, and 1 μg of mRNA with no caging agent bound thereto.

The absorbance of these samples was measured using a spectrophotometer (U3010, Hitachi, Ltd.). Figures 3(a) and (b) show the results. Figure 3(b) is an enlarged view of a part of Fig. 3(a). In Fig. 3(a) and (b), mRNA with no caging agent bound thereto is denoted as "control RNA" and mRNA with the caging agent bound thereto as "caged RNA."

As shown in Fig. 3(a) and (b), a second maximum peak and a third maximum peak at 333 nm and 383 nm, respectively, were observed for the mRNA with the caging agent bound thereto in addition to the major peak at 260 nm. The second and third maximum peaks both resulted from binding of the caging agent. The former peak corresponds to the absorbance maximum wavelength of the bound caging agent in the protonated state, and the latter peak corresponds to that of the bound caging agent in the ionated state. The results of absorbance spectrum analysis revealed that only about 40% of the caging agent bound to mRNA was ionated in a buffer (pH=7).

The number of molecules of the caging agent bound per mRNA molecule was calculated from the result. The number of molecules was calculated with the formula:

$$(1)/[(2) + (3)] = 34.8$$

(wherein (1) is a value obtained by dividing the absorbance (1.06) at 260 nm of mRNA with no caging agent bound thereto by 8,125$M^{-1}cm^{-1}$, (2) is a value obtained by dividing the absorbance at 383 nm of the mRNA with the caging agent bound thereto by 18,300 $M^{-1}cm^{-1}$, and (3) is a value obtained by the absorbance at 333 nm of the mRNA with the caging agent bound thereto by 11,100$M^{-1}cm^{-1}$). Therefore, it was assumed that the caging agent is bound per about 35 bases of mRNA molecule. Here, 8,125$M^{-1}cm^{-1}$ of (1) was calculated by the formula: $1.06 / (40 \times 1.06 \times 10^{-3} / 325) = 8,125$ (wherein 325 is the average molecular weight of adenin, guanine, cytosine and uracil, and 40 is the RNA concentration (μg/mL) when absorbance = 1); 18,300 $M^{-1}cm^{-1}$ of (2) is the molar absorption coefficient when the caging agent is ionated 100%; and 11,100$M^{-1}cm^{-1}$ of (3) is the molar absorption coefficient when the caging agent is protonated 100%. Based on these results, the amount of the bound caging agent (2) in the ionated state, and the amount of the bound caging agent (3) in the protonated state were calculated, and the sum ((2) + (3)) was used as the total binding number of the caging agent.

<u>In vitro translation</u>

An mRNA solution prepared by dissolving mRNA with the caging agent bound thereto in RNase-free water was apportioned to three samples, 1 to 3. A solution of mRNA with no caging agent bound thereto was prepared as sample 4.

These samples 1 to 4 were respectively apportioned onto the bottom surface of a 60-well microtiter plate (Nunclon Delta Surface, NUNC™, Nalge Nunc International). Subsequently, sample 2 was illuminated with ultraviolet rays at a wavelength of 365 nm using a 100W mercury lamp at 10% of the maximum intensity for 10 sec; and sample 3 was illuminated with ultraviolet rays at a wavelength of 365 nm under the same conditions for 20 sec. Samples 1 and 4 were not illuminated with ultraviolet rays. Next, an in vitro translation mixture (TNT Quick Coupled Transcription/ Translation System, Promega, U.S.A.) was added to these solutions, thereby synthesizing $^{35}$S labeled GFP.

After reaction, each sample was subjected to 12% SDS polyacrylamide gel electrophoresis for separation, and then analyzed by autoradiography using an image analyzer (BAS 5000, FUJIFILM, JAPAN). Figure 4 shows the results. In Fig. 4, lane 1 denotes sample 4, lane 2 denotes sample 3, lane 3 denotes sample 1, and lane 4 denotes sample 2.

As shown in Fig. 4, the translational activity of the mRNA with the caging agent bound thereto was significantly decreased (lane 2), while part of the translational activity of the mRNA was recovered by UV-illumination (lanes 3 and 4). These results show that binding of the caging agent to a nucleic acid molecule can suppress in vitro expression of mRNA, and detachment of the caging agent by UV-illumination can recover in vitro expression of mRNA.

[Example 2]

This example describes expression of GFP gene regulated in embryos of zebrafish (hereinafter, referred to as embryos) by applying the above-described method for regulating gene expression according to the present invention.

<u>Microinjection</u>

Similarly to Example 1, a caging agent and mRNA containing GFP gene was prepared, and then the caging agent was bound to the mRNA. Next, the mRNA with the caging agent bound thereto was prepared to have a concentration of 500 ng/μl, and then injected by microinjection to the embryo. Upon microinjection, UV light was eliminated from the illumination for a dissecting microscope using a UV cut film. The embryos injected were incubated in the dark at 28.5°C. In addition, under similar conditions, embryos to which mRNA with no caging agent bound thereto had been injected by microinjection were also prepared.

At 22 hours after fertilization, uniform and strong GFP signals were found in all the embryos (12 cells) injected with mRNA with no caging agent bound thereto (Fig. 5a and b). In contrast to this result, a significant decrease or quenching of GFP signals was found in all the 12 embryos injected with the mRNA with the caging agent bound thereto (Fig. 5c and d).

Photographs in Fig. 5 were taken using a microscope with a differential interference contrast optics (Axioplan II, Zeiss) connected to a high resolution digital camera (DP50, Olympus) or an epifluorescent dissecting microscope (MZFLIII, Leica) connected to a high sensitivity camera (C5810, Hamamatsu Photonics).

Activation by UV-illumination

Subsequently, to illuminate with ultraviolet rays embryos injected with the mRNA with the caging agent bound thereto, the embryos within the chorion were transferred into a small volume of water contained in V-shaped narrow grooves (1 mm in width, 0.5 mm in depth) curved on a Plexiglas plate. The orientation of the embryos was maintained by the surface tension of water. To prevent the embryos from drying, a maximum of 20 embryos were illuminated per cycle of UV-illumination. Thus, UV-illumination could be performed for 100 or more embryos within 30 min.

UV-illumination to the embryos was performed at 2 hours after fertilization or at the embryonic shield stage (6 hours after fertilization) using a band pass filter at 365 nm±6 nm (Filter Set 01, Carl Zeiss) for epifluorescent light, and a x20 dry objective lens (Plan Neofluar, Carl Zeiss) installed in a microscope (Axioskop II, Carl Zeiss). Duration of UV-illumination was regulated by opening and closing manually the path of the excitation light.

When illuminated with ultraviolet rays using a 100W mercury lamp (HBO 100W, Atto Arc, Carl Zeiss) at 10% of the maximum intensity for 10 sec, strong GFP fluorescence was observed in 5 out of 12 embryos, as shown in Fig. 5e and f. When illuminated at 10% of the maximum intensity for 20 sec, strong GFP fluorescence was observed in 7 out of 12 embryos, as shown in Fig. 5g and h. When illuminated at 20% of the maximum intensity for 10 sec, strong GFP fluorescence was observed in 8 out of 12 embryos, as shown in Fig. 5i and j. In addition, Fig. 6a shows an enlarged view of the typical example of embryos shown in Fig. 5d; Fig. 6b shows an enlarged view of the typical example of embryos shown in Fig. 5h; and Fig. 6c shows an enlarged view of the typical example of embryos shown in Fig. 5j.

As shown in Figs 5 and 6, GFP fluorescence was observed in embryos by UV-illumination, suggesting that translational activity of mRNA within the embryo was recovered by UV-illumination. The degree of recovery of translational activity differed depending on the conditions of UV-illumination, suggesting that conditions of UV-illumination should be appropriately adjusted to ensure detachment of a caging agent bound to mRNA.

Localized activation of mRNA by targeted spot UV-illumination

Next, embryos injected with mRNAs with the caging agents bound thereto were examined to verify that the mRNA could be locally activated by spot UV-illumination. To spot-illuminate with ultraviolet rays, the luminous field diaphragm for the epifluorescent light was closed as tight as possible so as to form the spot of ultraviolet rays. The spot of ultraviolet rays was illuminated specifically to the head or tail region of the embryos maintained within the chorion and positioned with their backs oriented to the V-shaped grooves, as shown in Fig. 7.

As shown in Fig. 8, GFP fluorescence could be observed in both the head and the tail regions at 22 hours after fertilization, confirming that translation of mRNA could be activated locally in both the head and tail regions. Figure 8a shows an example of the embryo for which GFP fluorescence could be observed locally at the head region. Figure 8b shows an example of the embryo for which GFP fluorescence could be observed locally at the tail region.

When only the head region side of the embryo was spot-illuminated with ultraviolet rays, GFP fluorescence could be observed only in the anterior part of the body of 23 out of 38 embryos. On the other hand, when only the tail region side of the embryo was spot-illuminated with ultraviolet rays, GFP fluorescence was observed only in the tail region side of 18 out of 39 embryos. Further, for the remaining embryos, as shown in Fig. 9a, although relatively enhanced intensity of GFP fluorescence was observed on the side spot-illuminated with ultraviolet rays, GFP fluorescence was distributed over the entire region. These results suggest that, as a whole, spatially limited expression of GFP protein was confirmed in about 70% of the embryos spot-illuminated with ultraviolet rays.

Further, to confirm that GFP fluorescence was induced by *de novo* synthesis of GFP protein, the embryos that were illuminated on the head region were stained with anti-GFP antibody. As shown in Fig. 9b, strong GFP immunoreactivity was observed for the embryo for which strong GFP fluorescence was found in the anterior part of the body at 22 hours after fertilization. Thus, it was confirmed that GFP fluorescence was induced by *de novo* synthesis of GFP protein.

[Example 3]

In Example 3, the effects of stability and specific expression concerning mRNA (hereinafter, En2 mRNA) containing a gene (hereinafter, En2 gene) encoding homeodomain transcription factor Engrailed-2 (hereinafter, En2) were examined by applying the method for regulating gene expression according to the present invention. En2 is expressed predominantly in the triangular region around the midbrain-hindbrain boundary (hereinafter, MHB), including the neighboring caudal midbrain and rostral hindbrain, from an early stage of embryonic development (13 hours after fertilization). Regarding En2, a model has been proposed based on experiments in chick embryos in which En2 acts as a transcriptional inhibitor of genes essential in the development of the brain region anterior to the midbrain.

Stability of En2 mRNA

Here, first, an embryo injected by microinjection with En2 mRNA with no caging agent bound thereto, and an embryo injected by microinjection with En2 mRNA with the caging agent bound thereto were prepared. Both mRNAs were prepared to have a concentration of 50 ng/μl, and then injected, about 50 pl each, into one-cell-stage embryos. In addition, preparation of a caging agent and binding of the caging agent to mRNA were performed in the same manner as in Example 1. Further, En2 mRNA was prepared in the same manner as employed for preparation of mRNA containing GFP protein in Example 1, except that mRNA was synthesized using a template which had been prepared by inserting the full length Engrailed-2 gene into a pCS2+ vector, followed by linearization with restriction enzyme Not I, or a template which had been prepared by inserting the full length Engrailed-2 gene into a pBluescriptKS vector, followed by linearization with restriction enzyme BamH I, and using mMESSAGE mMACHINE (Ambion, U.S.A.).

In the embryo injected with En2 mRNA with no caging agent bound thereto, a small reduction in eye size was observed in a 3-day old embryo, as shown in Fig. 10c and d. For comparison, Fig. 10a and b show photographs of the head region of a 3-day old embryo injected with no En2 mRNA.

When an embryo injected with En2 mRNA with the caging agent bound thereto was illuminated at the embryonic shield stage with ultraviolet rays, many embryos became completely eyeless, as shown in Fig. 10e and f. When an embryo injected with En2 mRNA with the caging agent bound thereto was not UV-illuminated, none or very few of the embryos showed the eyeless phenotype. In addition, UV-illumination was performed in the manner similar to Example 2 for 10 sec using a 100W mercury lamp (HBO 100W, Atto Arc, Carl Zeiss) at 10% of the maximum intensity.

Next, the effect of illuminating the head region with ultraviolet rays at 12 hours after fertilization following injection with En2 mRNA with the caging agent bound thereto was more quantitatively examined. For comparison, eye size was measured for the embryo injected with En2 mRNA with no caging agent bound thereto and for the embryo that was not injected with mRNA. Eye size measurement was performed at 44 hours after fertilization.

Fig. 11 shows the result. As shown in Fig. 11, while the average size of the eye was 215 μm in diameter in the embryos not injected with mRNA, it was 177 μm in diameter in the embryos injected with En2 mRNA with no caging agent bound thereto. In the embryos injected with En2 mRNA with the caging agent bound thereto, but not illuminated with ultraviolet rays, the average size of the eye was 201 μm in diameter. In contrast, it was 57 μm in diameter in the embryo injected with En2 mRNA with the caging agent bound thereto and illuminated with ultraviolet rays.

The results of Figs. 10 and 11 revealed that En2 mRNA was inactivated at 20 hours after fertilization when En2 mRNA with no caging agent bound thereto was simply injected, and a phenotype, such as a reduced eye size, was not significantly observed. In contrast, when injected with En2 mRNA with the caging agent bound thereto, translational activity was recovered by UV-illumination even at 17 hours after fertilization, suggesting that in vivo stability was significantly improved.

Further, mRNA (500 ng/μl) with the caging agent bound thereto containing GFP gene prepared in Example 2 and caged mRNA (50 ng/μl) containing En2 gene were co-injected into one-cell-stage embryos, and then the head regions were spot-illuminated with ultraviolet rays at 12 hours after fertilization. As a result, GFP fluorescence was observed in the affected forebrain region in the eyeless phenotype, as shown in Fig. 12a and b. This result indicates that both injected mRNAs were equally distributed within the embryos, and activated simultaneously by spot UV-illumination.

Moreover, expression of En2 within the embryos was confirmed by immunohistochemical staining using an anti-Engrailed protein monoclonal antibody (4D9). Figure 13 shows the result. As shown in Fig. 13a, expression of En2 was restricted to the region around the MHB in the embryos not injected with mRNA, whereas, as shown in Fig. 13b, in the affected eyeless embryos following injection of the caged mRNA and spot UV-illumination to the head region, ectopic overexpression of En2 was almost ubiquitously observed in the head region.

To confirm mRNA can be stabilized by binding of the caging agent, in situ hybridization was performed for the embryos injected with En2 mRNA with no caging agent bound thereto or those injected with En2 mRNA with the caging agent bound thereto, respectively, at 22 hours after fertilization using En2 probes. Thus, the presence of mRNA at 22 hours after fertilization could be confirmed. In the embryos injected with En2 mRNA with no caging agent bound thereto, as shown in Fig. 14a, at 22 hours after fertilization mRNA was expressed only in the MHB (indicated by an arrow in Fig. 14a), where endogenous En2 mRNA is normally expressed.

In contrast, in embryos injected with caged En2 mRNA and incubated in the dark, as shown in Fig. 14b, intense signals were detected throughout the whole body at 22 hours after fertilization. These results suggest that caged En2 mRNA is significantly more stable and persists longer than the native RNA when injected into one-cell-stage embryos.

[Example 4]

Example 4 shows an example of analyzing gene function using the method for regulating gene expression

according to the present invention. Specifically, the mechanism for inducing the eyeless phenotype by overexpression of En2 was examined by the method for regulating gene expression according to the present invention using En2 mRNA used in Example 3.

The expression patterns of two marker genes were examined by in situ hybridization: pax6, a marker gene for the diencephalic development, and krox20, a marker gene for development of the hindbrain,. A solution of caged En2 mRNA prepared in the same manner as in Example 2 was injected into embryos, and then the head of the embryo was spot-illuminated with ultraviolet rays at 12 hours after fertilization. Subsequently, the affected eyeless embryos were subjected to whole-mount in situ hybridization, thereby confirming the expression of pax6 and that of krox20.

Figure 15c shows the result of in situ hybridization performed for pax6. For comparison, Fig. 15d shows the result of in situ hybridization performed for pax6 using normal embryos. As shown in Fig. 15c and d, the pax6-positive region in the forebrain showed a dramatic reduction in size and shifted anteriorly because of overexpression of En2. Further, the krox20 positive region in the hindbrain remained unchanged both in the embryo injected with En2 mRNA and the normal embryo (not shown). In other words, expression of krox20 in the hindbrain was not affected by overexpression of En2. These results suggest that overexpression of En2 in the head region had not significantly affected development of the embryo at the level corresponding to or caudal to the hindbrain.

Next the expression patterns of four types of marker genes were similarly examined: emx1, a dorsal telencephalic marker gene; otx2, a dorsal midbrain marker gene whose caudal limit of expression shows a sharp edge near the caudal end of the midbrain; and pax2, and fgf8, marker genes of the MHB.

Figure 15b shows the result of in situ hybridization performed for emx1, and for comparison, Fig. 15a shows the result of in situ hybridization performed for emx1 using normal embryos. Further, Fig. 15f shows the result of in situ hybridization performed for otx2, and for comparison, Fig. 15e shows the result of in situ hybridization performed for otx2 using normal embryos. Furthermore, Fig. 15j shows the result of in situ hybridization performed for pax2, and for comparison, Fig. 15i shows the result of in situ hybridization performed for pax2 using normal embryos. Moreover, Fig. 14h shows the result of in situ hybridization performed for fgf8, and for comparison, Fig. 14g shows the result of in situ hybridization performed for fgf8 using normal embryos.

As shown in Fig. 15a and b, expression of emx1 in the telencephalon was completely lost by overexpression of En2. As shown in Fig. 15e and f, the otx2-positive region dramatically shifted rostrally, the anterior limit of which reached the most anterior tip of the brain because of overexpression of En2. As shown in Fig. 15g-j, expression of both pax2 and fgf8 was detected in the sharp narrow stripes in the MHB as in normal embryos, but it apparently shifted rostrally, because of overexpression of En2.

Next, double in situ hybridization was performed for otx2 and pax2, so that the expression of otx2 and that of pax2 were confirmed. Figure 16b shows the result of double in situ hybridization performed for otx2 and pax2. For comparison, Fig. 16a shows the result of double in situ hybridization performed for otx2 and pax2 using normal embryos. As shown in Fig. 16a and b, in the eyeless embryo due to overexpression of En2, the rostrally-shifted pax2-positive signal was still located adjacent to the caudal edge of the otx2-positive region as observed in the normal embryo.

Further, Fig. 17b shows the result of double in situ hybridization performed for otx2 and krox20. For comparison, Fig. 17a shows the result of double in situ hybridization performed for otx2 and krox20 using normal embryos. Overexpression of En2 resulted in a marked increase in the distances between the caudal edge of the otx2-positive region and krox20-positive stripes, and between the pax2-positive stripe and the otic vesicle, as shown in Fig. 17a and b, and Fig. 16a and b, respectively. These results suggest that overexpression of En2 causes expansion of the MHB itself.

The results shown in Figs. 15, 16 and 17 reveal that the head-specific induction of En2 at 12 hours after fertilization selectively caused a marked reduction in the forebrain, an anterior shift of the midbrain, and expansion of the MHB, while the structure posterior to the MHB remained intact. This result is consistent with a previous report in which overexpression of En2 downregulated pax6 in the chick diencephalon (Araki, I. and Nakamura, H., Development 126, 5127-5135 (1999)), but the forebrain was more significantly affected by overexpression of En2 using the method for regulating gene expression according to the present invention. This is probably because En2 was induced to higher levels in a broader region by the method for regulating gene expression according to the present invention.

As described above, the method for regulating gene expression according to the present invention can be applied when analyzing the function of a gene. The method for regulating gene expression according to the present invention can induce expression of a certain gene locally and at a desired time period, thereby enabling verification of the function of the gene.

As described in detail above, the present invention provides a method for regulating gene expression. With a caging agent having a certain structural formula, the present invention enables expression of a target gene site-specifically and with desired timing without damaging a cell itself or inducing mutation in a nucleic acid molecule within a cell. Therefore, the method for regulating gene expression according to the present invention is useful as it can perform determination and/or functional analysis of a novel gene which is specifically expressed spatially and temporally.

## Claims

1. A method for regulating gene expression which comprises illuminating with ultraviolet rays a nucleic acid molecule having a caging agent previously bound thereto and comprising a compound represented by the following formula (1):

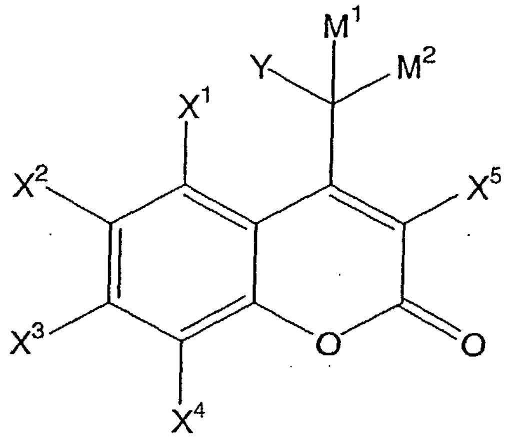

(1)

wherein $X^1$, $X^2$, $X^3$, $X^4$ and $X^5$ are independently -H, hydroxyl group, substituted alkoxy group, unsubstituted alkoxy group, -OC(O)$R^1$ group, -$NH_3$ group, -$NR^1R^2$ group, -$R^1$, -F, -Cl, -Br, -I, -COOH, -$NO_2$, -C(=O)$NHR^1$, -CN, -CHO, -C(=O)$R^1$ or -$SO_3H$; Y and $M^2$ are independently -H, -$R^1$, -$NR^1R^2$, -C(=O)$R^1$ or -COOH; $M^1$ is -H, -Cl, -Br, -I or -$OSO_2R^1$ or -OH, or M1 and $M^2$ form =$N_2$ or =$NNHR^1$; and R1 and R2 are independently substituted functional groups or unsubstituted functional groups selected from the group consisting of an alkyl group having a carbon number of 1 to 20, an alkenyl group having a carbon number of 2 to 20, an alkynyl group having a carbon number of 2 to 20, an alkoxy group having a carbon number of 1 to 20, a thioalkoxy group having a carbon number of 1 to 20, an alkylsulfonyl group having a carbon number of 1 to 20, an allylsulfonyl group having a carbon number of 4 to 16, a heteroalkyl group having a carbon number of 2 to 20, a heteroalkenyl group having a carbon number of 2 to 20, a cycloalkyl group having a carbon number of 3 to 8, a cycloalkenyl group having a carbon number of 3 to 8, an allyl group having a carbon number of 4 to 16, a heteroallyl group having a carbon number of 4 to 16, and a heterocyclyl group having a carbon number of 2 to 30.

2. The method for regulating gene expression according to claim 1 which comprises transfecting a nucleic acid molecule with said caging agent bound thereto into a host cell, and illuminating with said ultraviolet rays the transfected host cell.

3. The method for regulating gene expression according to claim 2 wherein said host cell allows penetration of long-wave ultraviolet rays.

4. The method for regulating gene expression according to claim 2 which comprises illuminating with said ultraviolet rays a certain region of said host cell, and allowing expression of a target gene only in the certain region.

5. The method for regulating gene expression according to claim 4 wherein said certain region is the entire host cell or a part of the host cell.

FIG.1

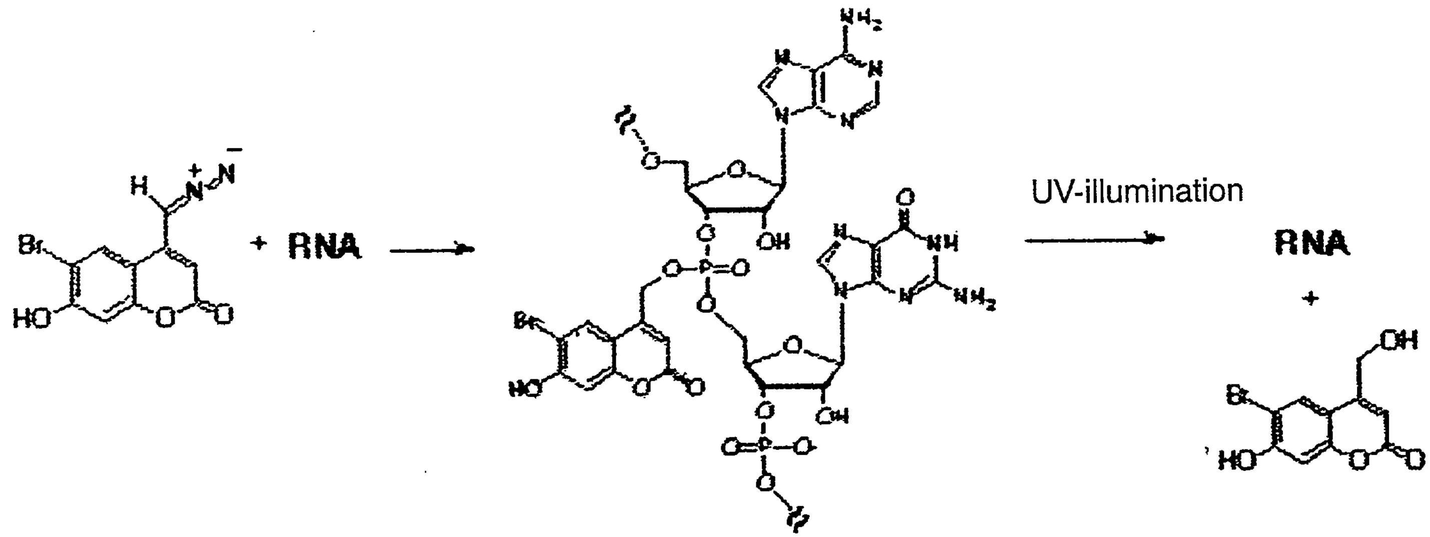

FIG.2

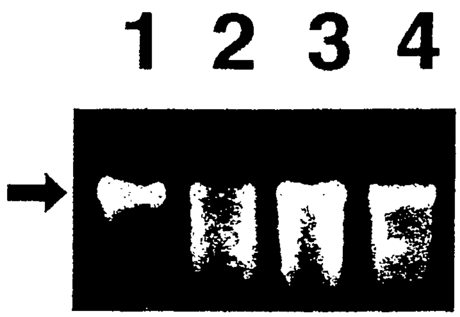

FIG.3

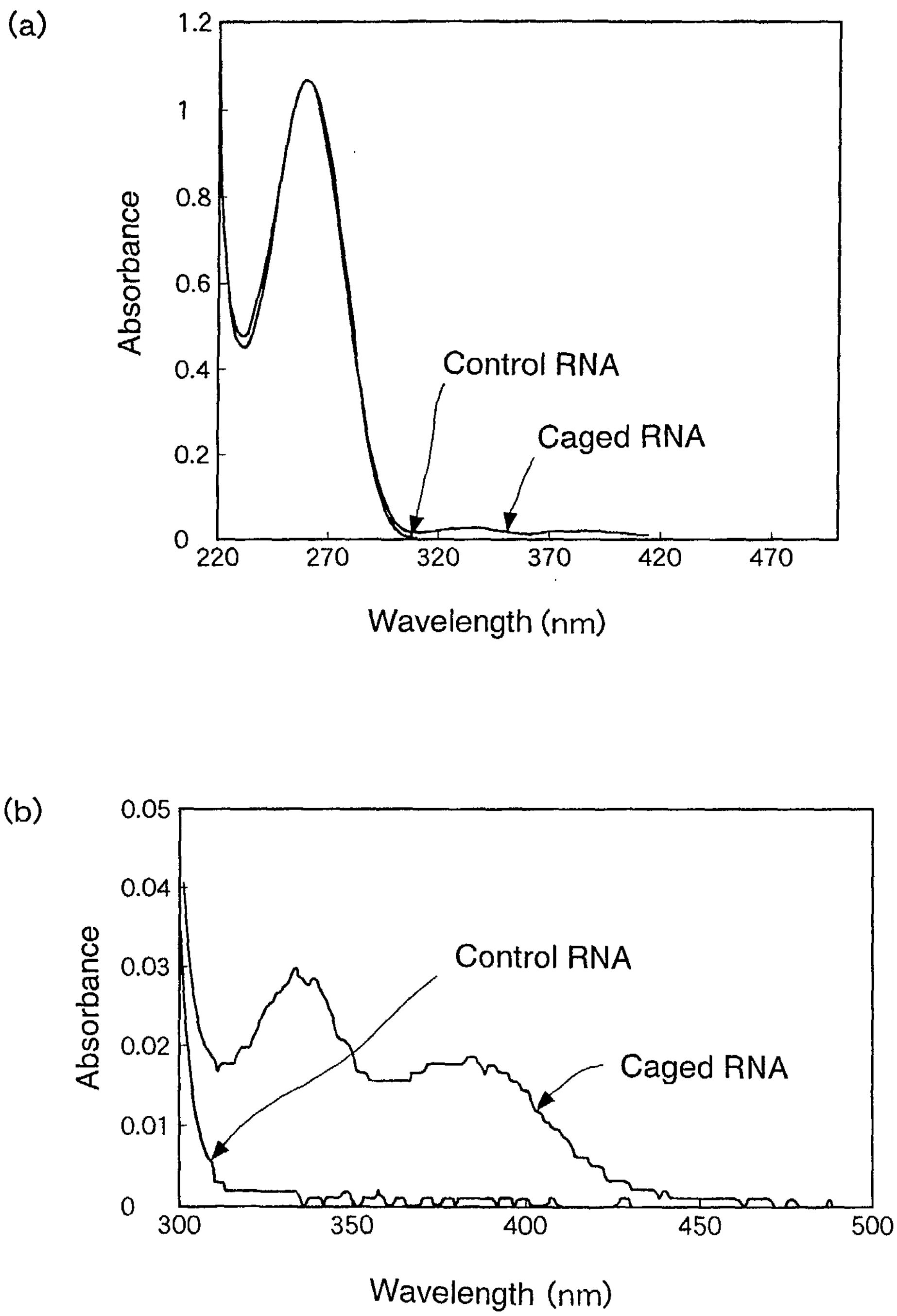
(a)
1.2
1
0.8
0.6
0.4
0.2
0
Absorbance
220
270
320
370
420
470
Wavelength (nm)
Control RNA
Caged RNA
(b)
0.05
0.04
0.03
0.02
0.01
0
Absorbance
300
350
400
450
500
Wavelength (nm)
Control RNA
Caged RNA

FIG.4

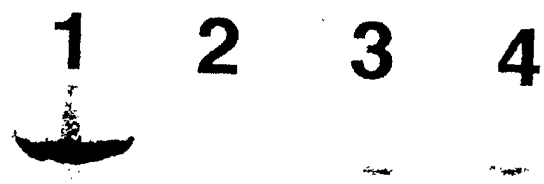

FIG.5

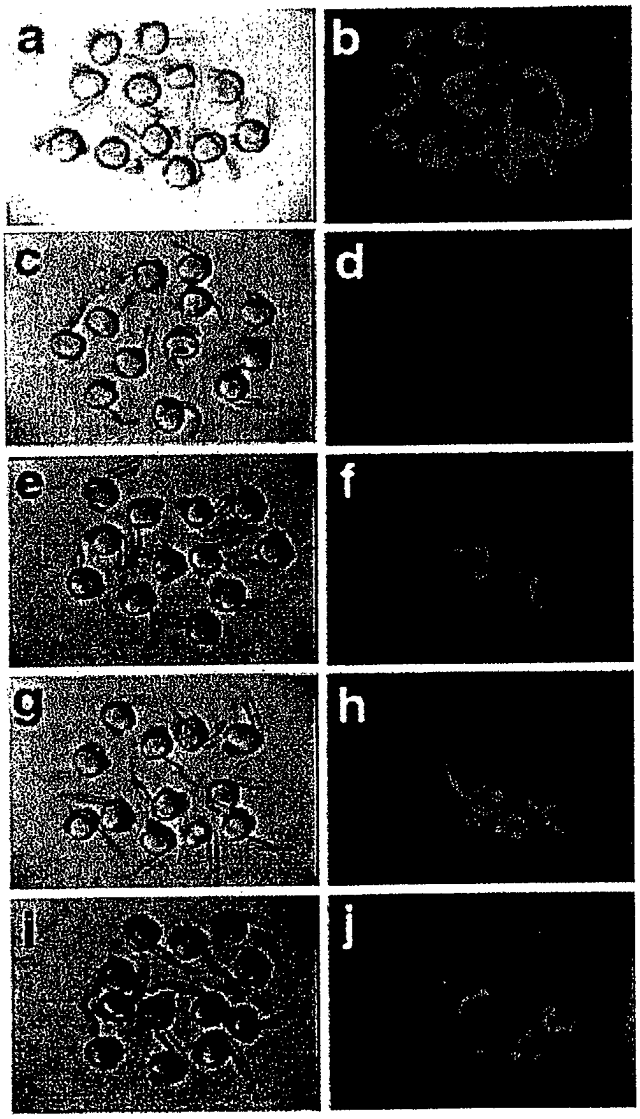

FIG.6

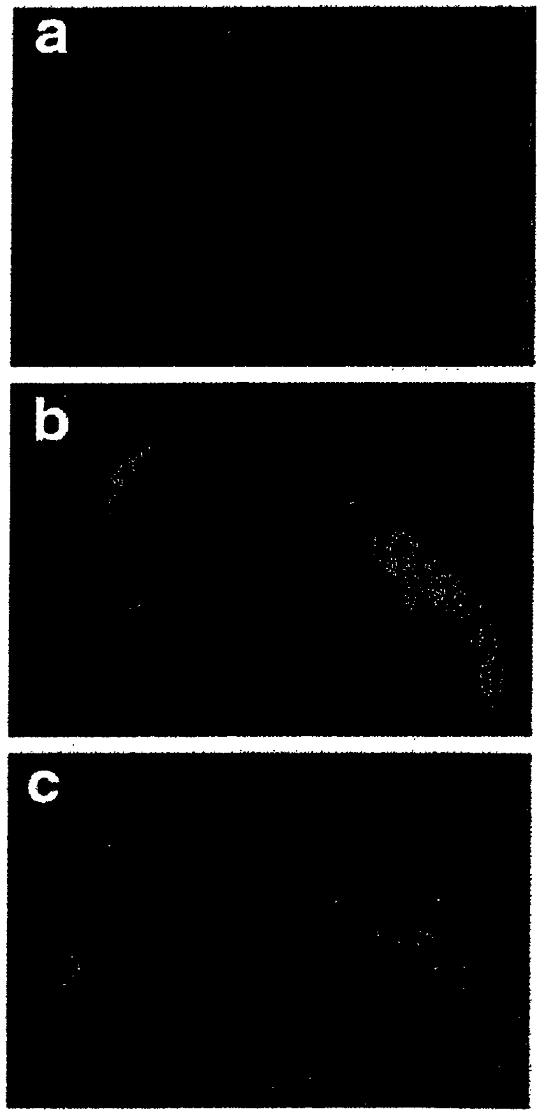

FIG.7

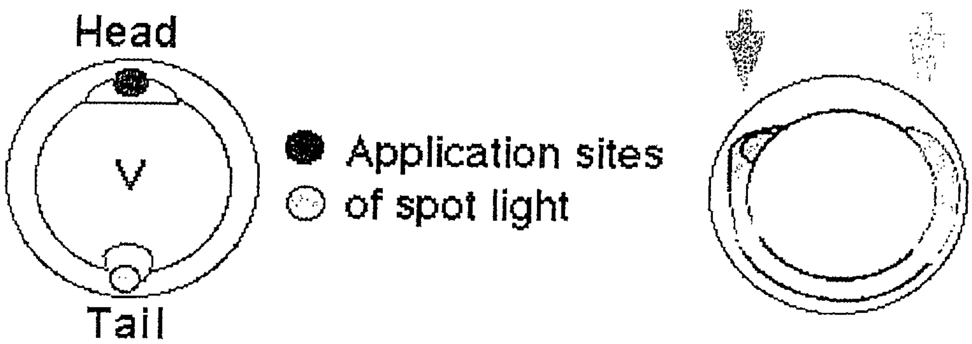

FIG.8

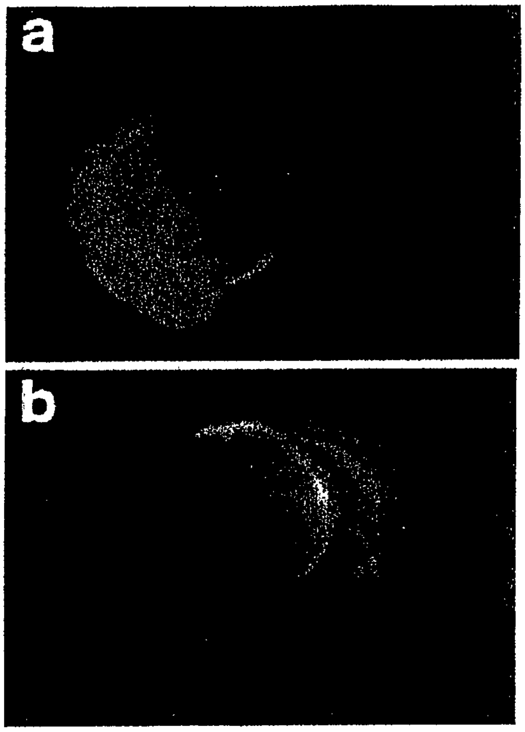

FIG.9

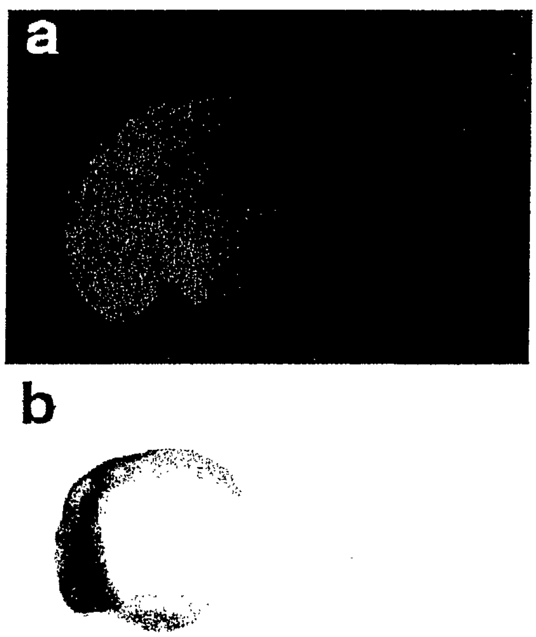

FIG.10

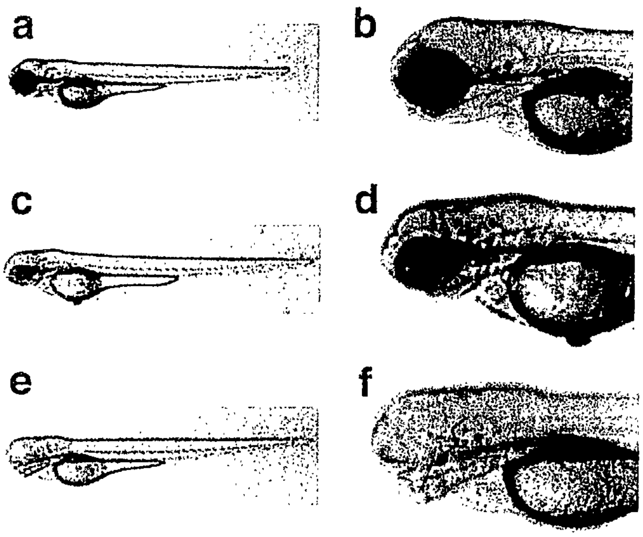

FIG.11

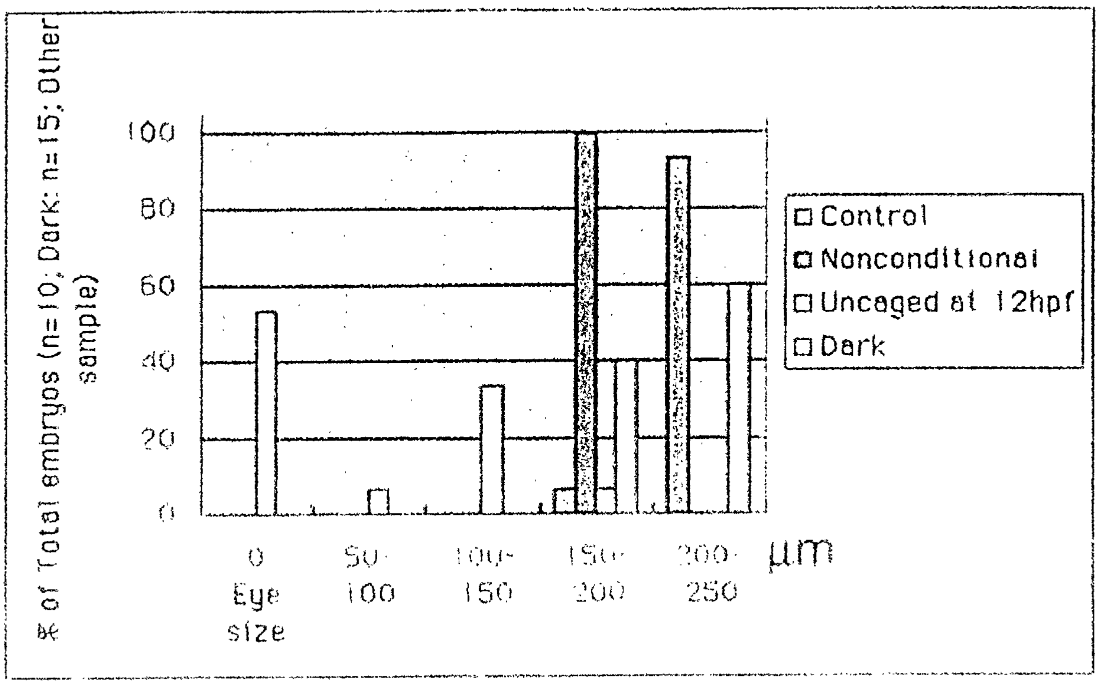
% of Total embryos (n=10; Dark: n=15; Other sample)
100
80
60
40
20
0
0 Eye size
50-100
100-150
150-200
200-250
µm
Control
Nonconditional
Uncaged at 12hpf
Dark

FIG.12

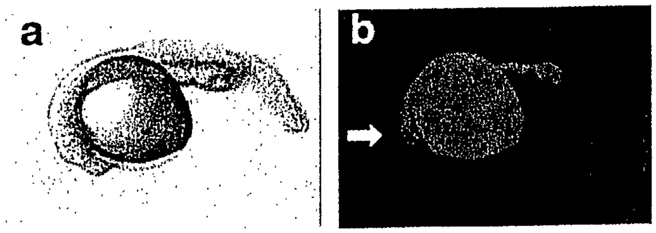


FIG.13

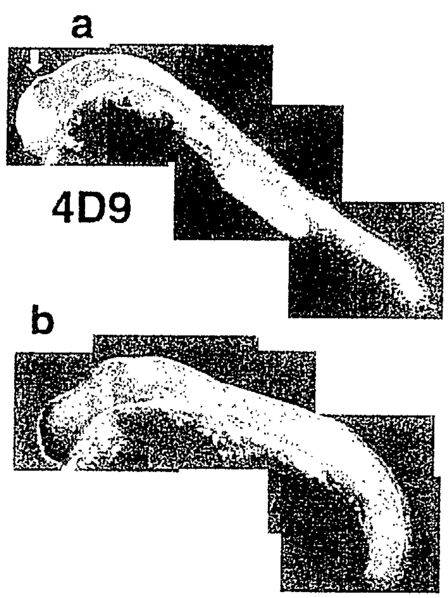


FIG.14

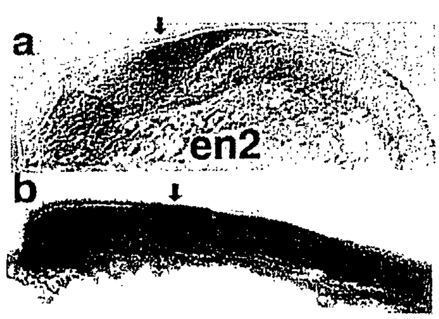

FIG.15

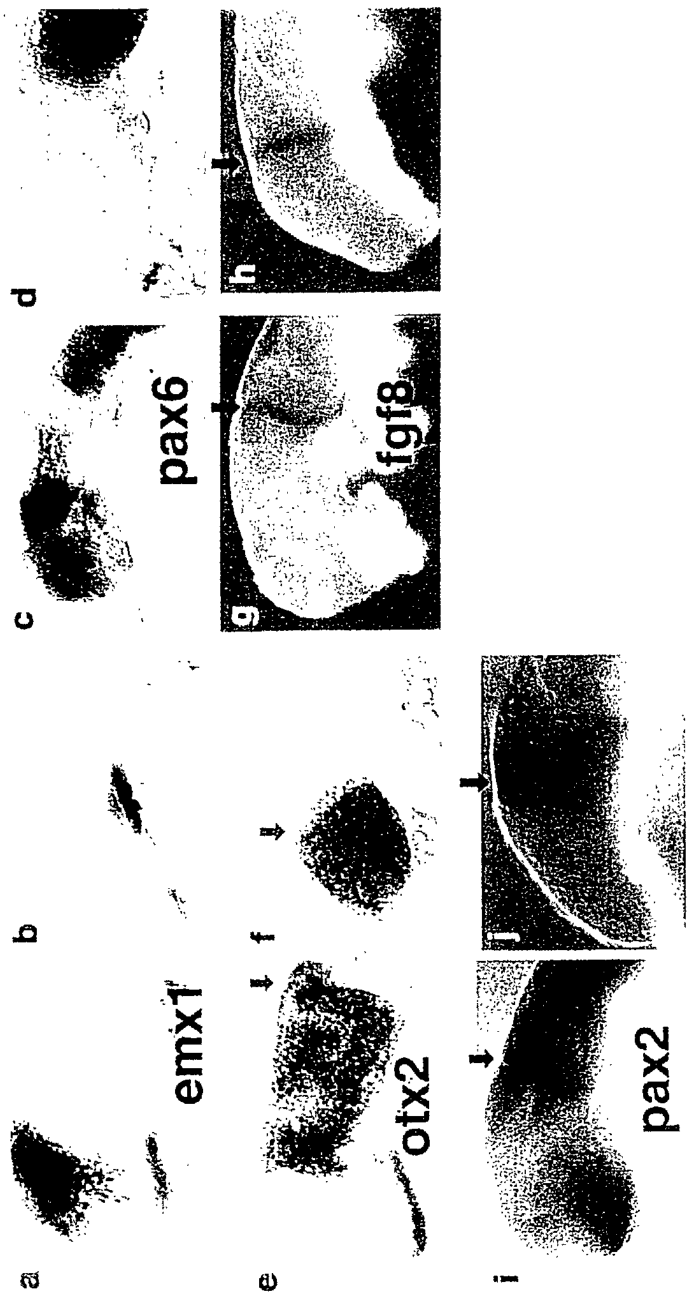

FIG.16

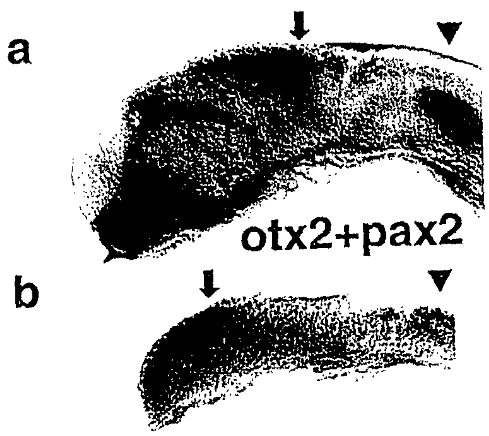


FIG.17

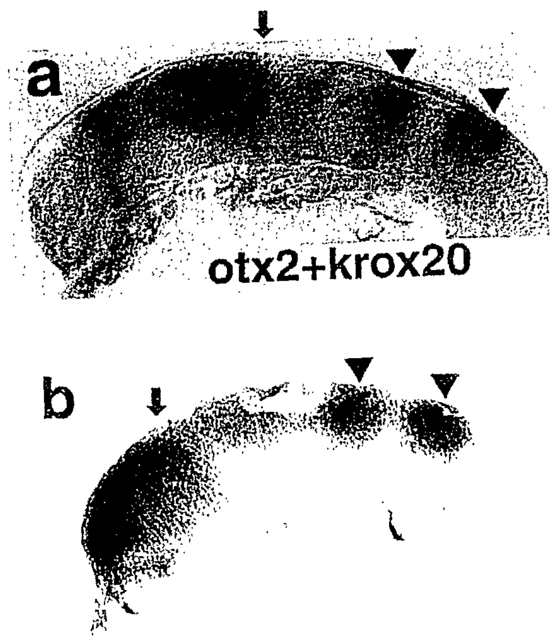

European Patent Office

# EUROPEAN SEARCH REPORT

Application Number
EP 02 25 2856

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 31588 A (UNIV CALIFORNIA)<br>2 June 2000 (2000-06-02)<br>* page 9, line 11 - line 18 *<br>* page 10, line 7 - line 25 *<br>* page 16, line 3 - line 7 *<br>* claims 62,63 * | 1 | C12N15/11<br>C07D311/10 |
| A | US 5 625 079 A (NERIO AILEEN ET AL)<br>29 April 1997 (1997-04-29)<br>* the whole document * | 1-5 | |
| P,X | ANDO H ET AL: "Photo-mediated gene activation using caged RNA/DNA in zebrafish embryos."<br>NATURE GENETICS. UNITED STATES AUG 2001, vol. 28, no. 4, August 2001 (2001-08), pages 317-325, XP002203447<br>ISSN: 1061-4036<br>* the whole document * | 1-5 | |

| TECHNICAL FIELDS SEARCHED (Int.Cl.7) |
|---|
| C12N<br>C07D |

The present search report has been drawn up for all claims

| Place of search | Date of completion of the search | Examiner |
|---|---|---|
| MUNICH | 25 June 2002 | Schwachtgen, J-L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 25 2856

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2002

| Patent document cited in search report | | Publication date | Patent family member(s) | | | Publication date |
|---|---|---|---|---|---|---|
| WO 0031588 | A | 02-06-2000 | US | 2002016472 | A1 | 07-02-2002 |
| | | | AU | 3102100 | A | 13-06-2000 |
| | | | EP | 1151350 | A1 | 07-11-2001 |
| | | | WO | 0031588 | A1 | 02-06-2000 |
| US 5625079 | A | 29-04-1997 | US | 5399719 | A | 21-03-1995 |
| | | | US | 5593823 | A | 14-01-1997 |
| | | | US | 6194139 | B1 | 27-02-2001 |
| | | | US | 5712085 | A | 27-01-1998 |
| | | | US | 5556993 | A | 17-09-1996 |
| | | | US | 6017691 | A | 25-01-2000 |
| | | | US | 2002006393 | A1 | 17-01-2002 |
| | | | US | 6218100 | B1 | 17-04-2001 |
| | | | US | 6004741 | A | 21-12-1999 |
| | | | US | 6004742 | A | 21-12-1999 |
| | | | US | 5871900 | A | 16-02-1999 |
| | | | AU | 699805 | B2 | 17-12-1998 |
| | | | AU | 7251194 | A | 17-01-1995 |
| | | | CA | 2165984 | A1 | 05-01-1995 |
| | | | EP | 0707476 | A1 | 24-04-1996 |
| | | | JP | 8512032 | T | 17-12-1996 |
| | | | US | 5972593 | A | 26-10-1999 |
| | | | WO | 9500141 | A1 | 05-01-1995 |
| | | | US | 5585503 | A | 17-12-1996 |
| | | | US | 5654443 | A | 05-08-1997 |
| | | | US | 5578736 | A | 26-11-1996 |

EPO FORM P0459

For more details about this annex : see Official Journal of the European Patent Office, No. 12/82